# EUROPEAN PATENT APPLICATION

(11) **EP 3 756 498 A1**
(43) Date of publication of application: **30.12.2020**
(21) Application number: 20182458.8
(22) Date of filing: 26.06.2020
(51) Int. Cl.: A42B 3/22, A42B 3/24, A61F 9/02

(54) **VISION IMPROVEMENT SYSTEM**

(30) Priority: 28.06.2019 GB 201909330
(71) Applicant: HD Inspiration Holding B.V., 1271 WP Huizen (NL)
(72) Inventor: ARNOLD, Derek Leslie, 1271 WP Huizen (NL)
(74) Representative: V.O.

(57) **Abstract**

The invention concerns a vision improvement system comprising: a visor (1), for example of a helmet, wherein the visor has an inward facing visor side (2) configured to face towards the eyes of a user of the visor, wherein the visor is configured to receive an insert (3) on the inward facing visor side;
a substantially transparent insert which is receivable by the visor on the inward facing visor side, wherein the insert has an inward facing insert side (4) configured to face towards the eyes of the user of the visor and an outward facing insert side (5) which is substantially opposite the inward facing insert side, wherein the insert includes at least a first positioning structure (6) for engaging at least a first positioning structure (7) of at least a first lens element (8, 16, 19, 20).

## Description

The present invention concerns a vision improvement system, in particular comprising a visor, for example of or for a helmet.

Fogging, i.e. condensation, is a known problem in the use of visors. This holds in particular for visors of helmets and the like, e.g. motor cycle helmets. Typically in such scenarios, visors have a protective function and are therefore composed of a hard material, which facilitates condensation on its surface. Helmets, in particular so-called full-face helmets, may limit ventilation at an inward facing visor side, where humidity may rise due to the user's respiration and transpiration. The fogging problem may be exacerbated by cooling of the visor due to a cold environment with respect to a temperature of an interior space of the helmet.

A known solution to this problem is the use of inserts, e.g. so-called pin-lock inserts (sometimes also called "pin-lock lenses", although the inserts themselves are generally not intended to provide optical lens functionality like focussing or dispersing light). Such an insert may be received at the interior side of the visor, i.e. the side which is facing towards the user's eyes during use. A received insert may generally be detachable and/or removable from the visor, e.g. for removing or replacing the insert and/or for cleaning the visor and/or the insert. The insert is typically substantially transparent, like the visor, but, contrary to the visor, the insert may be made of a substantially porous, hydrophilic material, so that condensation on the insert can be inhibited. The insert may further airtightly enclose an air layer with the interior side of the visor on which the insert is received, so that humid air is prevented from reaching that part of the visor. The result is that the user's vision through the insert and through the visor is substantially less affected by fogging.

As a residual problem, however, users of such visors with inserts may still experience fogging on surfaces of spectacle lenses or other vision improvement components which are positioned between the user's eyes and the insert. Spectacle lenses are typically made of a hard material, e.g. glass or polycarbonate, possibly with an antiscratch coating, and are thus prone to fogging in a similar way as the visor is. As a result, those users may choose not to wear their spectacles while wearing the visor, e.g. while riding a motor cycle, which behaviour may negatively affect road safety, among other potential problems.

While vision improvement elements like optical lenses could in theory be fixedly integrated in the visor itself, it is preferred that these elements are exchangeable, so that they can more easily be selected and/or replaced depending on a particular user's need, which may vary from other users' needs and which may change over time.

To this end, DE102012015506A1 discloses a vision protection device for a helmet including a visor unit having an inner visor and an outer visor arranged in front of the inner visor, characterized in that the visor unit includes at least one receiving device for receiving an optically effective lens unit. In an embodiment, a lens unit may be received in a space between the inner visor and the outer visor.

The receiving device of DE 102012015506A1 is formed as a recess in the inner or outer visor, wherein the recess has an inner contour that corresponds to a circumferential outer contour of the lens unit. Disadvantageously, adaptations by an optician may be required to fit the lens unit. Moreover, in case of substantial forces on the recess, e.g. due to driving on bumpy roads, the lens unit could be damaged and/or become dislodged from the recess in this configuration. Besides, the known system is relatively expensive in view of a relatively complex manufacturing process, and not very durable.

In an alternative embodiment of DE 102012015506A1, the lens unit is suspended at a user facing side by elastic bands, which can lead to disadvantageous shaking movements of the lens unit with respect to the visor unit, e.g. due to driving on a bumpy road, as well as fogging of the lens unit.

The present invention aims to overcome the above problems and disadvantages. In particular, the invention aims to provide a solution for vision improvement for visor users, wherein fogging is prevented and wherein lens elements can be easily, precisely and securely positioned and exchanged, e.g. by a visor user. Besides, the invention aims to provide improved durability, manufacturability and user safety.

Therefore, there is provided a vision improvement system comprising: a visor, for example of a helmet, wherein the visor has an inward facing visor side configured to face towards the eyes of a user of the visor, wherein the visor is configured to receive an insert on the inward facing visor side;
a substantially transparent insert which is receivable by the visor on the inward facing visor side, wherein the insert has an inward facing insert side configured to face towards the eyes of the user of the visor and an outward facing insert side which is faced away from the inward facing insert side, wherein the insert includes at least a first positioning structure for engaging at least a first positioning structure of at least a first lens element.

The positioning structures of the lens element and insert enable easy, secure and precise, preferably exchangeable, positioning of a lens element on the insert, while the lens element can be positioned e.g. on the outward facing insert side, i.e. away from an inward facing insert side, so that the lens element is less susceptible to fogging. The resulting configuration is safe for the user, in particular since the insert can provide a protective, preferably airtight, barrier between the lens element and the user during use. In particular, the positioning structures of the lens element and insert may enable that the lens element remain positioned on the insert while the insert is bent, e.g. to conform to a curvature of the visor, even when, for example, the lens element is substantially more stiff than the insert.

The system may further comprise at least a first lens element, for example having an inward facing lens side configured to face towards the eyes of the user of the visor, the lens element including at least a first positioning structure which is engageable with the at least first positioning structure of the insert.

By this configuration it is facilitated that the lens element is easily positioned in an appropriate orientation with respect to the user's eyes, e.g. with regard to its vision improvement properties such as optical lens properties. Inappropriate back-to-front positioning may be prevented in case e.g. an inward facing lens side includes the respective positioning structure.

The at least first positioning structure of the insert may e.g. include a socket, wherein the at least first positioning structure of the lens element includes a plug.

Such a plug-and-socket configuration provides means for easy, secure and precise exchangeable positioning. Moreover, such a plug may be easily included with a lens element during manufacturing of the lens element, e.g. during injection moulding. The skilled person will appreciate that the plug can be configured in various ways and shapes; preferably it is a substantially rigid plug element. It will also be appreciated that, according to a preferred embodiment (and e.g. using a certain flexibility of materials of the plug and/or socket), the plug may be somewhat oversized with respect to the socket in order to engage in a tight clamping fit, preferably an airtight fit, with the socket. For example, in case of a cylinder shaped plug, an external diameter of the plug may be slightly larger than a diameter of a respective socket that is to receive that plug.

The at least one positioning structure of the insert may be positioned substantially spaced apart from a central area of the insert, in order to reduce visual occlusion and/or distortion in the central area.

The lens element may include at least a second positioning structure which is engageable with at least a second positioning structure of the insert while the first positioning structure of the lens element is engaged with the first positioning structure of the insert.

Such second positioning structures may enable even more precise and secure positioning, in particular with respect to an orientation of the lens element.

The insert may be configured to receive further lens elements at respective further positions, wherein the lens elements may e.g. differ in terms of their vision improvement properties, e.g. optical lens properties. In this way, the vision improvement system can be easily adapted to specific and detailed vision improvement needs of a user.

According to a preferred embodiment, the insert includes at least one further first positioning structure which is configured to engage with at least one respective positioning structure of at least a second lens element. Preferably, the insert includes a sealing rim, wherein the insert is preferably configured to receive the at least second lens element at a position which is close to a lower section of the rim, and wherein the insert is preferably configured to receive the at first lens element at a position which is close to an upper section of the rim. According to a preferred embodiment, the second lens element has a vision improvement property which is substantially different from a respective vision improvement property of the first lens element.

According to a further embodiment, the vision improvement property is an optical lens property, for example a focal length or an optical power.

According to a further embodiment, the second lens element is configured for improving a vision regarding objects which are closer to the user, compared to a vision improvement provided by the first lens element.

According to a preferred embodiment, the at least first lens element and the at least second lens element together form at least a first lens element pair, wherein insert is configured to receive at least a second lens element pair while the first lens element pair is received on the insert, wherein the at least second lens element pair includes a respective first lens element and a respective second lens element, wherein the system is preferably configured so that, during use, the received lens elements of the at least first lens element pair, compared to the respective received lens elements of the at least second lens element pair, are closer to the user's left eye and further away from the user's right eye.

Further aspects of the invention provide an insert according to the features of claim 11, a lens element according to the features of claim 12 and a mould for producing a lens element according to the features of claim 13. In addition, the invention provides a method for improving the vision of a user of a visor according to the features of claim 14 and a method for manufacturing a lens element according to the features of claim 15.

Further advantageous elaborations of the invention are provided by the features of the dependent claims.

In the following, the invention will be explained with reference to exemplary embodiments and drawings. In the drawings:
Fig. 1a shows a front view of an insert according to a preferred embodiment;
Fig. 1b shows a partial cross section of the insert of Fig. 1a along the line I-I in Fig. 1a;
Fig. 1c shows a cross section of the insert of Fig. 1a along the line I'-I' in Fig. 1a;
Fig. 2a shows an exploded front view of an insert provided with lens elements according to a preferred embodiment;
Fig. 2b shows an exploded partial cross section of the insert provided with lens elements along the line II-II in Fig. 2a;
Fig. 2c shows an exploded cross section of the insert provided with lens elements along the line II'-II' in Fig. 2a;
Fig. 3a shows a front view of the insert provided with lens elements;
Fig. 3b shows a partial cross section of the insert provided with lens elements along the line III-III in Fig. 3a;
Fig. 3c shows a cross section of the insert provided with lens elements along the line III'-III' in Fig. 3a;
Fig. 4 shows a schematic midsagittal cross section of an exemplary configuration of a visor and an insert provided with lens elements in front of eyes of a user;
Fig. 5a shows a top view of an exemplary mould for producing a lens element, having positioning structures positioned therein;
Fig. 5b shows a cross section of the mould and positioning structures along the line V-V in Fig. 5a; and
Fig. 5c is similar to Fig. 5b, showing the mould including a formed lens element.

Corresponding or similar features are denoted by corresponding or similar reference signs.

In the drawings, transparent elements may be drawn as opaque to help distinguish different elements and spatial configurations.

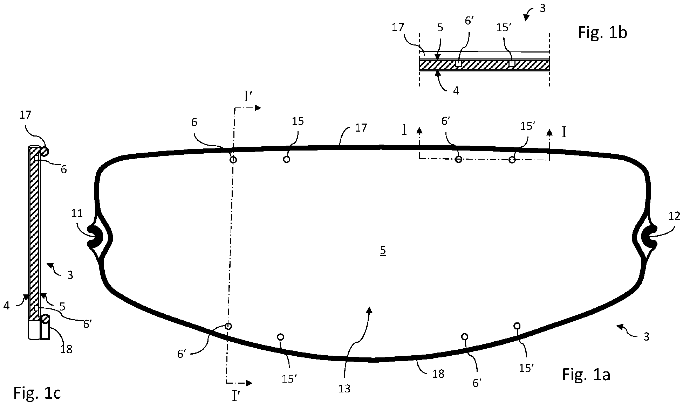

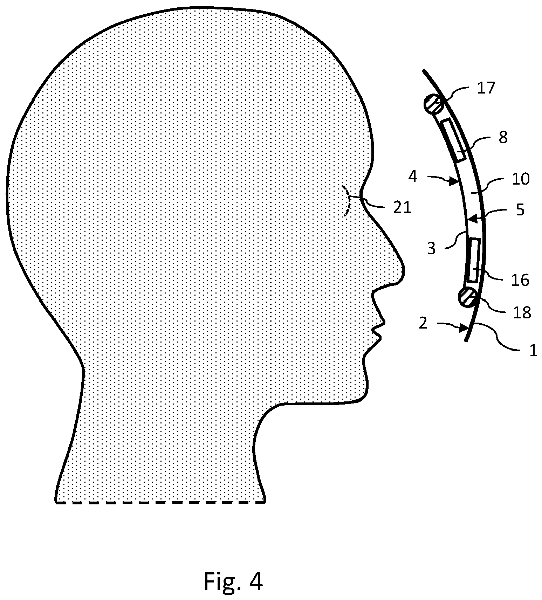

The drawings schematically show a vision improvement system according to a preferred embodiment, wherein the system comprises a visor 1 (see Fig. 4), for example of a helmet, wherein the visor 1 has an inward facing visor side 2 configured to face towards the eyes 21 of a user of the visor 1, wherein the visor 1 is configured to receive an insert 3, for example a pin-lock insert 3, on the inward facing visor side 2. A helmet having a pin-lock visor is known as such, see e.g. Fig. 1 of DE102012015506A1, or www.pinlock.com.

The insert 3 is substantially transparent for providing the user with a substantially unobstructed view of at least a part of the user's surroundings through the visor. In some embodiments, the insert 3 may be coloured, for example to filter light passing through the insert 3, for example to reduce glare from bright light sources such as the sun. Exemplary insert colouring schemes include smoke, dark smoke, orange and yellow colours. The insert 3 may be photochromic, i.e. it may be configured such that its colouring is dependent on exposure to light. In the present embodiment, the insert 3 mainly consists of a relatively thin (transparent) plate, e.g. having a thickness smaller than 2 mm, for example a thickness of 0.6 mm, 0.8 mm or 1.15 mm, or another thickness.

To accommodate various shapes and sizes of visors 1 and/or their users, the insert 3 may be produced in corresponding various shapes and sizes, and/or the insert 3 may be adaptable with respect to its shape and/or size.

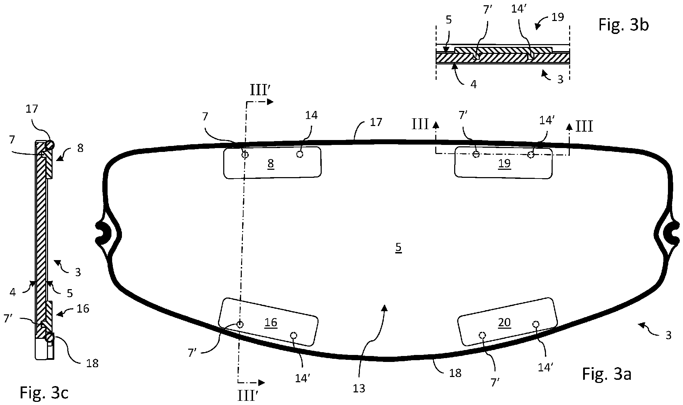

In Figs. 1a, 1b, 1c, 2a, 2b, 2c, 3a, 3b, 3c, the insert 3 is drawn as substantially flat, i.e. not curved. It will be appreciated that the insert 3 may be curved after mounting and/or be bendable along its surface to substantially match one or more respective curvatures of the visor 1, as shown for example in Fig. 4.

The insert 3 may include one or more pin-lock visor connectors 11, 12 (see Fig. 1a) for (preferably detachably) connecting the insert 3 to the visor 1, for example to respective one or more pin-lock connectors (not shown) of the visor 1.

With further reference to Fig. 4, the insert 3 has an inward facing insert side 4 which is configured to face towards the eyes 21 of the user and an outward facing insert side 5.

The insert 3 preferably includes a sealing rim, e.g. including an upper rim section 17 and a lower rim section 18 (see the drawings). During use, the lower rim section 18 may be positioned lower than the upper rim section 17. The rims 17, 18 may be included in and/or together form a continuous rim 17, 18 which substantially encompasses the insert 3 or which substantially extends along a contour of the insert 3. The rim 17, 18 may be, for example, of a silicone material, preferably a flexible and dry (hardened) silicone material, and is preferably configured to provide a sealing connection between the insert 3 and the visor 1. In this way (as is shown in Fig. 4), an air space 10 between the insert 3 and the visor 1 can be substantially airtightly closed off from an environment (i.e. when the visor 1 is provided with the insert 3). Such a configuration can advantageously prevent that humid air reaches the thus sealed off part of the inward facing visor side 2, thus enabling to prevent condensation, for example of water, on the inward facing visor side 2.

To enable prevention of condensation on the inward facing insert side 4, at least a part and preferably substantially all of the inward facing insert side 4 is substantially hydrophilic, for example by including a hydrophilic membrane material or other porous material.

In a preferred example, the outward facing insert side 5 is configured to receive at least a first lens element 8 and preferably a second lens element 16. During use, as shown in Fig. 4, such lens elements 8, 16 may thus be advantageously enclosed within the closed off space 5, so that humid air can be prevented from reaching the lens elements 8, 16 and from causing condensation on their surfaces.

The lens elements 8, 16 are drawn as substantially straight and flat, but they may be curved and their thickness may vary across their surface, e.g. to form an optical lens (in particular to provide a non-zero optical power, after mounting via the insert 3 to the visor 1)). Alternatively, for example, the lens element can be a Fresnel lens. Moreover, the lens elements 8, 16 may be flexible or bendable, e.g. to substantially conform to a curvature of the insert 3 after mounting the insert 3 on a curved visor 1.

A thickness of the rim 17, 18 may be configured depending on a thickness, e.g. a maximum thickness, of the lens elements 8, 16. For example, a thickness of the rim 17, 18 may be larger than a thickness of the lens elements 8, 16 in order to prevent that a lens element 8, 16 comes into contact with the visor 1 when the insert 3 is received on the visor 1. In this way, the system may be configured to space the lens element 8, 16 apart from the visor 1. Advantageously, preventing such contact may help to prevent optical distortion due to pressure exerted on the lens element 8, 16.

The lens elements 8, 16 are preferably configured to enable improved vision of the user, for example by each including an optical lens having a focal length or optical power corresponding to a vision improvement need of the user. Lens elements may be produced in a variation of vision improvement properties to address a variation of user needs. By providing the insert 3 with appropriately selected one or more lens elements, an appropriate vision improvement system for the particular user may thus be configured.

Lens elements 8, 16 may be configured to prevent condensation on their surfaces, e.g. by including a substantially hydrophilic material, e.g. in a coating layer of the lens elements.

As shown in the drawings, the outward facing insert side 5 preferably includes at least a first positioning structure 6 for engaging at least a first positioning structure 7 of at least the first lens element 8. The thus engageable at least first positioning structure 7 is preferably included in an inward facing lens side 9 of the at least first lens element 8.

In a preferred embodiment, the at least first positioning structure 6 of the insert 3 includes a socket 6, wherein the at least first positioning structure 7 of the inward facing lens side 5 includes a plug 7 (in this case protruding substantially normally from that side 5), to be received in/by the socket 6. In this example, the socket 6 is depicted as a blind bore in the insert 3. Alternatively, the socket 6 may protrude through the entire insert 3 (e.g. provided by a perforation or bore in the insert 3). In the latter case it is preferred that the plug 7 of a respective lens element can airtightly seal the socket 6 after mounting.

In this way, a simple and robust configuration is provided which is suitable for various types of visors and inserts, for example including a range of visor shapes and sizes.

A lens element 8 including such a plug 7 can be easy to produce, for example using a moulding process, e.g. plastic injection moulding.

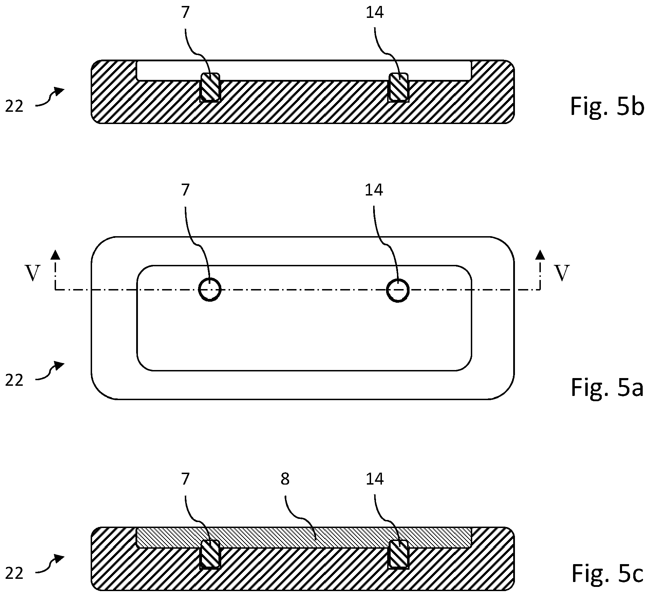

Fig. 5a shows an exemplary mould part 22 for producing an exemplary lens element 8 using moulding according to an exemplary method, wherein the mould 22 is injected or filled with a molten material, for example a polycarbonate resin. In a preferred method, the at least first positioning structure 7 (and possibly a spaced-apart second positioning structure 14) is positioned at least partially in the mould 22 before and/or during and/or after injecting the molten material (but before hardening), thus bringing the positioning structure 7 (and optional second structure 14) in contact with the molten material. By subsequently hardening the injected material, each positioning structure 7, 14 may be firmly bound to the lens element which is thus being formed, as in Fig. 5c.

It should be observed that each lens element can be made of various materials. For example, good results can be achieved in case the lens element is substantially, preferably entirely, composed of a material or compound including or consisting of: an aliphatic material, for example a moisture absorbent material, for example including at least one of polyurethane, polycarbonate, cellulose acetate and cellulose propionate.

In some embodiments, the at least first positioning structure 7 (and possibly other positioning structures) may be substantially composed of the same material as another part of the lens element 8, for example the rest of the lens element 8. In this way, the positioning structure may form a single piece with the other part 8, so that production, e.g. using injection moulding, can be simplified.

The at least one positioning structure 6 of the insert, and possibly other positioning structures 15, 6', 15' may be substantially spaced apart from a central area 13 of the insert 3, for example substantially adjacent to an edge (17, 18) of the insert 3. This can prevent that the users vision is adversely affected by the positioning structures. Also, depending on specific user and/or application needs, it may be preferred to thus position the at least first lens element 8 substantially away from the central area 13, for example to limit the vision improvement function of the lens element 8 to a more peripheral area.

To further improve ease and stability of positioning, in particular regarding an orientation of the lens element 8, the inward facing lens side 9 may include at least a second positioning structure 14 which is engageable with at least a second positioning structure 15 of the insert while the first positioning structure 7 of the inward facing lens side is engaged with the first positioning structure 6 of the insert. The respective first and second positioning structures 6, 7, 14, 15 are thus positioned in accordance with a desired position and orientation of the lens element 8 relative to the insert 3.

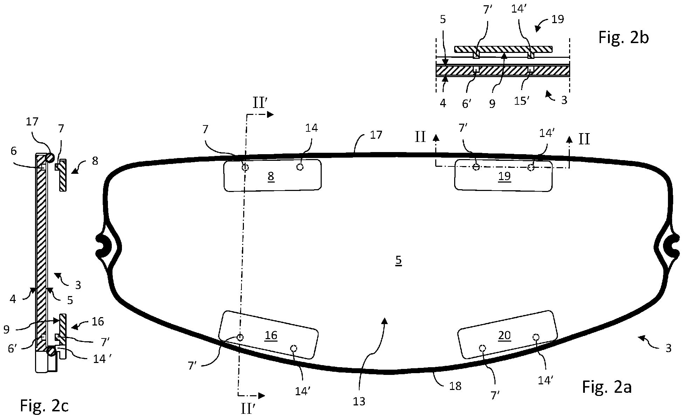

As shown in Fig. 2a and 4, the system may include multiple lens elements 8, 16, 19, 20 which may be used simultaneously, so that the user's vision may be improved in multiple parts of their field of vision, with vision improvement properties that may vary among the multiple parts. In the present, preferred example, four spaced-apart lens elements are present.

For example, the outward facing insert side 5 may include at least one further first positioning structure 6' which is configured to engage with at least one respective positioning structure 7' of at least a second lens element 16. The positioning structures 7, 7' thus impose at least partially the respective positions at which the respective lens elements 8,16 can be received by the insert 3. For example, as in the drawings, the insert 3 may thus be configured to receive the at least second lens element 16 at a position which is closer to the lower edge 18 compared to a position of the received first lens element 8, so that, during use, the second lens element 16 is lower than the first lens element 8. Such a configuration may enable a different selection of vision improvement properties for a lower part of the user's field of vision compared to a higher part.

In a particularly advantageous exemplary embodiment, the second lens element 16, which is positioned closer to the lower edge 18, has a different focal length or optical power compared to the first lens element 8, wherein the focal length or optical power of the second lens element 16 is selected to improve the user's vision regarding objects which are closer to the user, for example for reading a document in the user's hand or a dial or display of a vehicle, e.g. a motor cycle.

The first and second lens elements may thus form a first lens element pair 8, 16. For users with different eyes, it may be preferred to use a separate, e.g. different lens element pair for each eye, wherein each lens element pair is positioned appropriately with respect to each respective eye. As shown in the drawings, in a preferred embodiment, the outward facing insert side is configured to receive at least a second lens element pair 19, 20 while the first lens element pair 8, 16 is received on the outward facing insert side, wherein the at least second lens element pair 19, 20 includes a respective first lens element 19 and a respective second lens element 20. For users with a left eye and a right eye, the first lens element pair 8, 16 may be positioned closer to, e.g. substantially in front of, the user's left eye, while the second lens element pair 19, 20 may be positioned closer to, e.g. substantially in front of, the user's right eye.

In this way, there is provided a solution for vision improvement for visor users, wherein fogging is prevented and wherein lens elements can be easily, precisely and securely positioned and exchanged, e.g. by a visor user. Besides, the solution provides improved durability, manufacturability and user safety.

While the invention has been explained with reference to exemplary and preferred embodiments, it will be appreciated that variations of these are possible and are considered to be included in the scope of the invention, defined by the claims. For example, the insert may be configured to receive any positive number of lens elements at any positive number of positions and/or orientations. Each lens element may be provided with any positive number of positioning elements. A lens element may include an optical lens, but may alternatively or additionally also be configured to improve vision in another way, e.g. by filtering light. Positioning structures may be plugs or sockets or other mutually engaging structures. Lens elements may be produced by various methods, for example injection moulding or machining, and each lens element may be composed of one or more components, wherein components may include positioning structures.

Positioning structures, in particular sockets, of the insert may extend e.g. from the outward facing insert side to various depths in the insert, including up to and including the inward facing insert side.

Appropriate alternative materials to those described in the examples may be used. Shapes and dimensions, in particular of the visor, insert, insert rims, lens elements and positioning structures, are not limited to those in the drawings.

Further, it is preferred that the lens element can be positioned onto said outwardly facing insert side. Alternatively, the lens element can be positioned onto the inner insert side (facing a user during operation).

### List of reference signs

- 1.: Visor
- 2.: Inward facing visor side
- 3.: Insert
- 4.: Inward facing insert side
- 5.: Outward facing insert side
- 6.: First positioning structure of insert
- 6'.: Further first positioning structure of insert
- 7.: First positioning structure of first lens element
- 7'.: First positioning structure of further lens element
- 8.: First lens element
- 9.: Inward facing lens side
- 10.: Space between visor and insert
- 11.: First visor connector of insert
- 12.: Second visor connector of insert
- 13.: Central area of insert
- 14.: Second positioning structure of first lens element
- 14'.: Second positioning structure of further lens element
- 15.: Second positioning structure of insert
- 15'.: Further second positioning structure of insert
- 16.: Second lens element
- 17.: Upper rim section of insert
- 18.: Lower rim section of insert
- 19.: First lens element of second lens element pair
- 20.: Second lens element of second lens element pair
- 21.: Eye of user
- 22.: Mould part

## Claims

1. A vision improvement system comprising:
- a visor (1), for example of a helmet, wherein the visor has an inward facing visor side (2) configured to face towards the eyes (21) of a user of the visor, wherein the visor is configured to receive an insert (3) on the inward facing visor side (2);
- a substantially transparent insert (3) which is receivable by the visor on the inward facing visor side, wherein the insert has an inward facing insert side (4) configured to face towards the eyes of the user of the visor and an outward facing insert side (5) which is faced away from the inward facing insert side of the insert, wherein the insert (3) includes at least a first positioning structure (6, 6', 15, 15') for engaging at least a first positioning structure (7, 7', 14, 14') of at least a first lens element (8, 16, 19, 20).

2. A vision improvement system according to claim 1, wherein the system further comprises:
- at least a first lens element (8, 16, 19, 20) including at least a first positioning structure (7, 7', 14, 14') which is engageable with the at least first positioning structure of the insert.

3. A vision improvement system according to claim 2, wherein the at least first lens element (8, 16, 19, 20) has an inward facing lens side (9) configured to face towards the eyes of the user of the visor, wherein the inward facing lens side includes the at least first positioning structure (7, 7', 14, 14').

4. A vision improvement system according to any of claims 1-3, wherein the at least first positioning structure of the insert includes a socket (6, 6', 15, 15'), wherein the at least first positioning structure of the inward facing lens side includes a plug (7, 7', 14, 14').

5. A vision improvement system according to any of the preceding claims, wherein the visor and the insert are configured to substantially airtightly close off a space (10) therebetween, wherein the system is preferably configured to space the at least first lens element (8, 16, 19, 20) apart from the visor (1), wherein the at least first lens element (8, 16, 19, 20) is preferably located in the substantially airtightly closed off space (10).

6. A vision improvement system according to any of the preceding claims, wherein the insert is configured to prevent condensation, for example of water, on a surface of the insert, wherein the surface of the insert is substantially hydrophilic, wherein the surface includes at least a part, preferably more than half, preferably substantially all of the inward facing insert side (4).

7. A vision improvement system according to any of the preceding claims, wherein the at least one positioning structure is made in one piece with the respective lens element (8,16,19,20).

8. A vision improvement system according to any of the preceding claims, wherein the at least one positioning structure of the insert is positioned substantially spaced apart from a central area (13) of the insert.

9. A vision improvement system according to any of the preceding claims, wherein the lens element includes at least a second positioning structure (14, 14') which is engageable with at least a second positioning structure (15, 15') of the insert while the first positioning structure is engaged with the first positioning structure of the insert.

10. A vision improvement system according to any of the preceding claims, wherein the insert includes at least one further first positioning structure (6', 15') which is configured to engage with at least one respective positioning structure (7', 14') of at least a second lens element (16, 20).

11. An insert of a system according to any of the preceding claims.

12. A lens element of a system according to any of claims 1-10, wherein the lens element is preferably substantially, preferably entirely, composed of a material or compound including or consisting of: an aliphatic material, for example a moisture absorbent material, for example including at least one of polyurethane, polycarbonate, cellulose acetate and cellulose propionate.

13. A mould (21) for producing, for example by injection moulding, a lens element according to claim 12.

14. A method for improving the vision of a user of a visor, the method comprising:
- providing a system according to any of claims 1-10;
- engaging the at least one positioning structure of the at least one lens element with a respective positioning structure of the insert, thus attaching the at least one lens element to the insert, and preferably to the outward facing insert side;
- attaching the insert provided with the at least one lens element to the visor at the inward facing visor side; and
- providing the user with the visor.

15. A method for manufacturing a lens element according to claim 12, the method comprising:
- providing a mould according to claim 13;
- positioning the at least first positioning structure at least partially in the mould;
- filling the mould with a molten material, for example including a resin; and
- hardening the injected material, for example by cooling and/or irradiating, thereby forming the lens element and binding the at least first positioning structure to the lens element.
